# FASCICULE DE BREVET EUROPEEN

(11) **EP 2 579 970 B1**
(45) Date de publication et mention de la délivrance du brevet: **30.07.2014**
(21) Numéro de dépôt: 11792003.3
(22) Date de dépôt: 07.06.2011
(51) Int. Cl.: B01F 11/00, G01N 21/84, G01N 21/51

(54) **DISPOSITIF ET PROCEDE DESTINE A MESURER LES PROPRIETES D'UN MILIEU COMPLEXE PAR UNE ANALYSE DE L'EVOLUTION DE LA LUMIERE RETRODIFFUSEE ET/OU TRANSMISE**
VORRICHTUNG UND VERFAHREN ZUR MESSUNG DER EIGENSCHAFTEN EINES KOMPLEXEN MEDIUMS DURCH ANALYSE DES UNTERSCHIEDS IN RÜCKGESTREUTEM UND/ODER DURCHFALLENDEM LICHT
DEVICE AND METHOD INTENDED TO MEASURE THE PROPERTIES OF A COMPLEX MEDIUM BY ANALYSIS OF THE VARIATION IN BACKSCATTERED AND/OR TRANSMITTED LIGHT

(30) Priorité: 09.06.2010 FR 1054556
(43) Date de publication de la demande: 17.04.2013
(73) Titulaire: Centre National de la Recherche Scientifique (CNRS), 75016 Paris (FR)
(72) Inventeur: COUNORD, Jean-Louis, F-92500 Rueil-Malmaison (FR); DUFAUX, Jacques, F-91700 Sainte Genevieve des Bois (FR)
(74) Mandataire: Derambure, Christian
(86) Numéro de dépôt international: PCT/FR2011/051293
(87) Numéro de publication internationale: WO 2011/154655

(56) Documents cités:
- DE-U1- 20 112 276
- FR-A1- 2 383 444
- US-A1- 2007 064 521

## Description

L'invention concerne le domaine de la mesure des propriétés d'un milieu complexe.

L'invention se rapporte à un dispositif et un procédé destiné à mesurer les propriétés d'un milieu complexe par une analyse de l'évolution de la lumière rétrodiffusée et/ou transmise par ce milieu complexe après une étape préalable d'agitation.

Un tel dispositif est, à titre d'exemple non limitatif, utilisé pour mesurer un temps d'agrégation et/ou un index de sédimentation d'un milieu complexe, tel qu'une suspension sanguine.

Par exemple, l'analyse de la vitesse d'agrégation d'une suspension sanguine permet d'identifier l'élévation de l'agrégation du sang chez des patients atteints de pathologies telles que le diabète, l'hypertension, les accidents vasculaires cérébraux, la stase du retour veineux, le glaucome, etc.

En effet, la viscosité du sang dépend de nombreux paramètres, dont l'agrégabilité des globules rouges ainsi que la composition en macromolécules du plasma. L'agrégabilité des globules rouges qui est un phénomène réversible, dépend des conditions d'écoulement. La taille des agrégats formés peut atteindre 50 à 100 µm dans les zones de bas cisaillements du réseau microcirculatoire. Dans ces zones, plus particulièrement du coté veinulaire, les agrégats se rassemblent au centre du vaisseau créant, près de la paroi, une couche de plasma vide de globules ce qui favorise une diminution de la viscosité apparente de la suspension sanguine. Mais cette diminution ne peut s'exercer que si le niveau d'agrégation n'est pas trop élevé et que les agrégats peuvent se détruire au passage des capillaires terminaux de diamètre inférieur à la taille de l'agrégat, là où le cisaillement augmente. Au contraire, lorsque l'agrégation est trop élevée, la circulation est ralentie voir même bloquée. La connaissance de l'agrégation dans le sang est donc utile pour le médecin cherchant à prévenir certains risques hémorhéologiques.

L'invention concerne, selon un premier de ses aspects, un dispositif destiné à mesurer les propriétés d'un milieu complexe par une analyse de l'évolution de la lumière rétrodiffusée et/ou transmise par le milieu complexe lors d'une phase de mesure postérieure à une étape préalable d'agitation, ce milieu complexe comprenant des agrégats et étant contenu dans un conteneur s'étendant selon une direction longitudinale. A cet égard, le dispositif de mesure comporte des moyens de réception aptes à recevoir et à supporter le conteneur ; des moyens de maintien aptes à maintenir ce conteneur dans une position fixe ou sensiblement fixe vis-à-vis des moyens de réception lorsque ces moyens de réception sont animés d'un mouvement d'agitation provoquant la destruction des agrégats dudit milieu complexe ; des moyens de support d'un module de mesure de la lumière rétrodiffusée et/ou transmise par ledit milieu complexe, les moyens de support étant aptes à s'associer structurellement avec le module de mesure de sorte à le supporter et agencés vis-à-vis desdits moyens de réception de manière à permettre au module de mesure d'émettre des rayons lumineux d'émission de sorte à illuminer ledit milieu complexe et de recevoir des rayons lumineux rétrodiffusés et/ou transmis par ledit milieu complexe à tout moment de la phase de mesure lorsque ce module de mesure est associé structurellement aux moyens de support.

Un tel dispositif est connu de l'état de la technique, notamment par l'exemple qu'en donne le document « RBC Laserdifractometry and RBC Aggregation with a rotational Viscometer: Comparison with Rheoscope and Myrenne aggrometer », M.R. Hardeman, R.M. Bauersachs, H.J. Meiselman. Clin. Hemorheol. En effet, ce document décrit un dispositif basé sur la mesure de la lumière rétrodiffusée par le milieu complexe que constitue la suspension sanguine.

Son principe est le suivant. La suspension sanguine est éclairée par un faisceau lumineux de sorte que plus les globules rouges sont agrégés plus l'intensité lumineuse rétrodiffusée est faible, la surface éclairée étant plus petite. La suspension sanguine - constituant le milieu complexe - est placée entre deux cylindres coaxiaux dont l'un peut tourner rapidement afin de soumettre les agrégats à un cisaillement suffisant pour les désagréger. Lorsque le cylindre tournant est brutalement arrêté, les agrégats se reforment plus ou moins rapidement. Le temps d'agrégation est alors l'un des paramètres permettant de caractériser la capacité d'agrégation des globules rouges à partir de l'analyse de courbes représentant l'évolution temporelle de l'intensité de la lumière rétrodiffusée par le milieu complexe.

Toutefois, la mise en oeuvre de ce dispositif présente plusieurs inconvénients. En premier lieu, il est nécessaire, pour effectuer la mesure, d'ouvrir les tubes contenant les échantillons de sang afin d'introduire la suspension sanguine entre les deux cylindres coaxiaux. Or, la législation mise en place ces dernières années concernant la manipulation du sang humain a imposée de nombreuses consignes de sécurité rendant ce type de manipulations irréalisables. En outre, le nettoyage de l'espace de faible épaisseur compris entre les deux cylindres coaxiaux est particulièrement délicat et nécessite un temps de préparation d'environ 15 minutes par échantillon. De même, le temps préconisé pour réaliser la désagrégation des agrégats pour réaliser la mesure est de plusieurs minutes, ce qui est prohibitif pour des mesures de routines. Enfin, le système mécanique complexe consistant à faire tourner l'un des cylindres coaxiaux par rapport à l'autre est de coût relativement élevé.

Il est également connu de l'état de la technique, le document WO-A-2008/072870 qui décrit un appareil de mesure du taux d'agrégation d'une suspension sanguine. Cet appareil comprend notamment un conteneur pour recevoir la suspension sanguine et un agitateur à intégrer dans le conteneur pour désagréger les agrégations contenues dans cette suspension sanguine de manière à obtenir les conditions initiales de la mesure ainsi qu'une unité d'induction permettant de mettre en mouvement l'agitateur.

Comme précédemment, cet appareil présente plusieurs inconvénients. En effet, les dimensions du conteneur doivent nécessairement être adaptées à la réception d'un agitateur, ce qui implique généralement de transférer la suspension sanguine d'un tube de prélèvement sanguin à un conteneur particulier. En outre, il est indispensable d'introduire l'agitateur à l'intérieur du conteneur ce qui provoque des problèmes de sécurité contraignantes compte tenu des normes législatives précitées concernant la manipulation des prélèvements sanguins. Il convient également de souligner que la désagrégation obtenue grâce à l'agitateur n'est pas optimale.

Dans un domaine différent qui est celui des agitateurs destinés à mélanger du sang avec un liquide anti-coagulant, il est également connu le document FR-A- 2 501 057. Celui-ci présente un agitateur de sang en poches présentant un plateau oscillant dont le mouvement est imprimé par trois galets formant un plan incliné. Trois bras porte galets sont fixés sur un plateau tournant comportant une roue dentée qui engrène sur un pignon fixé à l'arbre de sortie d'une réducteur de vitesse entraîné par un moteur électrique. Des dispositifs analogues sont encore exposés dans les documents FRA-2383444, DE 20112 276 U1 et US 2007/064521.

A nouveau, ce dispositif présente des inconvénients. Il ne permet pas de réaliser une agitation suffisante de la poche pour obtenir une désagrégation des agrégats contenue dans le sang. Et, en tout état de cause, l'agitation doit être prolongé pendant une durée importante qui n'est pas adaptée aux mesures de routine.

Dans ce contexte, l'invention à pour but de proposer un dispositif et un procédé destinés à mesurer les propriétés d'un milieu complexe qui soit exempt de l'une au moins des limitations précédemment évoqués.

Plus particulièrement, il existe un besoin non satisfait pour un dispositif et un procédé destinés à mesurer les propriétés d'un milieu complexe par une analyse de l'évolution de la lumière rétrodiffusée et/ou transmise par le milieu complexe lors d'une phase de mesure postérieure à une étape préalable d'agitation qui permette de réaliser une désagrégation optimum des agrégats dans un intervalle de temps minimum, sans nécessiter l'ouverture du conteneur recevant le milieu complexe à analyser.

A cet effet, le dispositif et le procédé selon l'invention, par ailleurs conforme à la définition générique qu'en donne le préambule ci-dessus, est essentiellement caractérisé en ce qu'il comporte des moyens de mise en mouvement aptes à imposer auxdits moyens de réception un mouvement d'agitation comprenant au moins un déplacement de va-et-vient selon un vecteur directionnel s'étendant en proportion non négligeable selon la direction longitudinale, de manière à provoquer des contraintes de cisaillement dans le milieu complexe engendrant la destruction des agrégats dudit milieu complexe lorsque ledit conteneur est reçu et supporté par les moyens de réception.

Grâce à cet agencement, il est possible d'imposer à un conteneur tel qu'un tube de prélèvement sanguin un mouvement capable d'engendrer des contraintes de cisaillement suffisamment élevées pour désagréger les agrégats contenu dans le milieu complexe dans un intervalle de temps limité. De façon surprenante, il a en effet été constaté que ce mouvement - semblable au geste d'une infirmière agitant sensiblement dans sa longueur un tube de prélèvement sanguin - permet d'obtenir dans un bref délai un taux de désagrégation particulièrement élevé. En outre, une telle configuration permet d'utiliser une suspension sanguine directement prélevée d'un patient sans nécessiter de transfuser cette suspension sanguine depuis le tube de prélèvement sanguin vers un conteneur spécialement prévu à cet effet. *A forfiori,* l'étape de nettoyage du conteneur est également supprimée et, après analyse, le tube de prélèvement peut être réutilisé sans que l'état de la suspension sanguine n'ait été modifié.

Il convient de noter que s'entend d'un « vecteur directionnel s'étendant en proportion non négligeable selon la direction longitudinale » tout vecteur directionnel présentant une composante longitudinale - correspondant à une projection du vecteur directionnel dans la direction longitudinale du conteneur lorsqu'il est en position dans les moyens de réception - suffisante pour désagréger les agrégats du milieu complexe. De préférence, cette composante longitudinale est supérieure à toute autre composante transversale s'étendant selon une direction perpendiculaire à la direction longitudinale susmentionnée.

Il convient également de noter que le terme de « milieu complexe » vise tout type de milieu qui, lorsqu'il est soumis a des contraintes de cisaillement, change de structure ce qui engendre la destruction d'agrégats ou autre types d'amas et permet un retour à un état initial reproductible.

Selon une réalisation, les moyens de mise en mouvement sont aptes à imposer auxdits moyens de réception un mouvement d'agitation comprenant au moins un déplacement de va-et-vient selon une translation unidirectionnelle.

Selon une réalisation, les moyens de mise en mouvement sont aptes à imposer auxdits moyens de réception un mouvement d'agitation comprenant au moins un déplacement de va-et-vient selon une translation circulaire autour d'un axe de rotation.

Selon une réalisation, les moyens de mise en mouvement sont aptes à imposer auxdits moyens de réception un mouvement d'agitation comprenant au moins un déplacement de va-et-vient selon une rotation autour d'un axe de rotation. Notamment, l'axe de rotation peut être positionné à une distance sensiblement comprise entre 50 et 200 millimètres du centre de gravité des moyens de réception et le déplacement de va-et-vient autour de l'axe de rotation peut être sensiblement compris entre 10° et 90°.

Il a été constaté que de tels mouvements de rotation suffisent à engendrer très simplement de fortes contraintes de cisaillement à l'intérieur du conteneur et ainsi à obtenir un taux élevé de désagrégation.

Selon une réalisation, les moyens de mise en mouvement comprennent un moteur présentant un arbre de rotation, un bras de levier coopérant avec l'arbre de rotation et relié aux moyens de réception de sorte que les moyens de mise en mouvement sont aptes à entraîner les moyens de réception en déplacement autour de l'arbre de rotation.

Selon une réalisation, les moyens de maintien sont formés par joints toriques recouvrants une portion de la surface intérieure des moyens de réception. Ainsi, le conteneur peut être maintenu aux moyens de réception par simple adhérence aux joints toriques.

Selon une réalisation, les moyens de maintien sont formés par un pince ou une pièce de butée déformable ou non.

Selon une réalisation comprenant le module de mesure de la lumière rétrodiffusée et/ou transmise par ledit milieu complexe, celui-ci comporte une source lumineuse apte à émettre les rayons lumineux d'émission en direction dudit milieu complexe de sorte à illuminer ledit milieu complexe, et un détecteur optique apte à recevoir les rayons lumineux rétrodiffusés et/ou transmis par ledit milieu complexe en réponse à l'illumination dudit milieu complexe.

Selon une réalisation, la source lumineuse émet un rayonnement monochromatique.

Selon une réalisation, les moyens de support du module de mesure sont associés structurellement et fonctionnellement aux moyens de réception du conteneur de sorte que le mouvement d'agitation imposé aux moyens de réception est également imposé au module de mesure. Une cohérence entre le module de mesure et les moyens de réception est ainsi obtenue, ce qui permet d'assurer la position relative du module de mesure vis-à-vis du milieu complexe contenu dans le conteneur.

Selon une réalisation, les moyens de support du module de mesure sont dissociés structurellement des moyens de réception de sorte que ces moyens de support sont en mouvement relatif vis-à-vis de ces moyens de réception lorsque lesdits moyens de réception sont en mouvement d'agitation.

Selon une réalisation, les moyens de réception présentent une fenêtre agencée vis-à-vis des moyens de support de sorte que les rayons lumineux d'émission et les rayons lumineux rétrodiffusés sont aptes à traverser cette fenêtre lors de la phase de mesure.

Selon un autre aspect, l'invention concerne également un procédé destiné à mesurer les propriétés d'un milieu complexe par une analyse de l'évolution de la lumière rétrodiffusée et/ou transmise par le milieu complexe lors d'une phase de mesure postérieure à une étape préalable d'agitation, ce milieu complexe comprenant des agrégats et étant contenu dans un conteneur s'étendant selon une direction longitudinale, le procédé comprenant une pluralité d'étapes successives durant lesquelles :
■ le conteneur est reçu et supporté par des moyens de réception ;
■ le conteneur est maintenu dans une position fixe ou sensiblement fixe vis-à-vis des moyens de réception ;
■ la phase de mesure consistant à émettre des rayons lumineux d'émission de sorte à illuminer le milieu complexe et recevoir des rayons lumineux rétrodiffusés et/ou transmis par ledit milieu complexe.

Plus particulièrement, selon l'invention l'étape préalable d'agitation du conteneur consiste à :
■ mettre les moyens de réception dans un mouvement d'agitation comprenant au moins un déplacement de va-et-vient selon un vecteur directionnel s'étendant en proportion non négligeable selon la direction longitudinale,
■ de manière à provoquer des contraintes de cisaillement dans le milieu complexe engendrant la destruction des agrégats dudit milieu complexe lorsque ledit conteneur est reçu et supporté par les moyens de réception.

Selon une réalisation, l'étape préalable d'agitation consiste à mettre les moyens de réception et le conteneur dans un mouvement d'agitation comprenant au moins un déplacement de va-et-vient selon une translation unidirectionnelle.

Selon une réalisation, l'étape préalable d'agitation consiste à mettre les moyens de réception et le conteneur dans un mouvement d'agitation comprenant au moins un déplacement de va-et-vient selon une translation circulaire autour d'un axe de rotation.

Selon une réalisation, l'étape préalable d'agitation consiste à mettre les moyens de réception et le conteneur dans un mouvement d'agitation comprenant au moins un déplacement de va-et-vient selon une rotation autour d'un axe de rotation.

Selon une réalisation, l'axe de rotation est positionné à une distance sensiblement comprise entre 50 et 200 millimètres du centre de gravité des moyens de réception.

Selon une réalisation, le déplacement de va-et-vient autour de l'axe de rotation est sensiblement compris entre 10° et 90°.

Selon une réalisation, le conteneur est maintenu par un joint torique recouvrant une portion de la surface interne des moyens de réception.

Selon une réalisation, le conteneur est maintenu par un pince ou une pièce de butée déformable ou non.

Selon une réalisation, durant la phase de mesure une source lumineuse émet les rayons lumineux d'émission en direction dudit milieu complexe de sorte à illuminer ledit milieu complexe, un détecteur optique reçoit les rayons lumineux rétrodiffusés et/ou transmis par ledit milieu complexe en réponse à l'illumination dudit milieu complexe.

Selon une réalisation, la source lumineuse émet un rayonnement monochromatique.

Selon une réalisation, les moyens de support du module de mesure sont associés structurellement et fonctionnellement aux moyens de réception du conteneur de sorte que le mouvement d'agitation imposé aux moyens de réception est également imposé au module de mesure.

Selon une réalisation, les moyens de support du module de mesure sont dissociés structurellement des moyens de réception de sorte que ces moyens de support sont en mouvement relatif vis-à-vis de ces moyens de réception lorsque lesdits moyens de réception sont en mouvement d'agitation.

Selon une réalisation, les moyens de réception présentent une fenêtre transparente vis-à-vis des moyens de support du module de mesure de sorte que les rayons lumineux d'émission et les rayons lumineux rétrodiffusés traversent cette fenêtre lors de la phase de mesure.

Selon une réalisation, le module de mesure transmet des données collectées durant la phase de mesure à une unité d'analyse déportée structurellement ou non.

Selon une réalisation, l'unité d'analyse utilise les données collectées pour calculer la vitesse de sédimentation dudit milieu complexe postérieurement à la phase de mesure.

D'autres caractéristiques et avantages de l'invention ressortiront clairement de la description détaillée qui en est faite ci-après, à titre indicatif et nullement limitatif, en référence aux dessins annexés, dans lesquels :
- la figure 1 représente une vue en perspective d'un dispositif destiné à mesurer la vitesse d'agrégation d'une suspension sanguine contenue dans un tube de prélèvement sanguin par une analyse de l'évolution de la lumière rétrodiffusée par la suspension sanguine lors d'une phase de mesure postérieure à une étape préalable d'agitation ;
- la figure 2 représente une vue en perspective du dispositif de la figure 1 dans lequel le module de mesure de la lumière est structurellement et fonctionnellement délié des moyens de réception du tube de prélèvement sanguin ;
- la figure 3 représente une vue en perspective du dispositif de la figure 1 dans lequel le module de mesure de la lumière est structurellement et fonctionnellement lié aux moyens de réception du tube de prélèvement sanguin et ce dernier est maintenu en position dans les moyens de réception ;
- la figure 4 représente une vue en perspective du dispositif de la figure 3, dans lequel les moyens de mis en mouvement déplacent les moyens de réception du tube de prélèvement sanguin selon une rotation autour d'un axe de rotation ;
- les figures 5a et 5b représentent une vue schématique d'un autre mode de réalisation du dispositif selon l'invention dans lequel le mouvement d'agitation correspond à un déplacement selon une translation unidirectionnelle ; et
- les figures 6a et 6b représentent une vue schématique d'un autre mode de réalisation du dispositif selon l'invention dans lequel le mouvement d'agitation correspond à un déplacement selon une translation circulaire autour d'un axe de rotation.

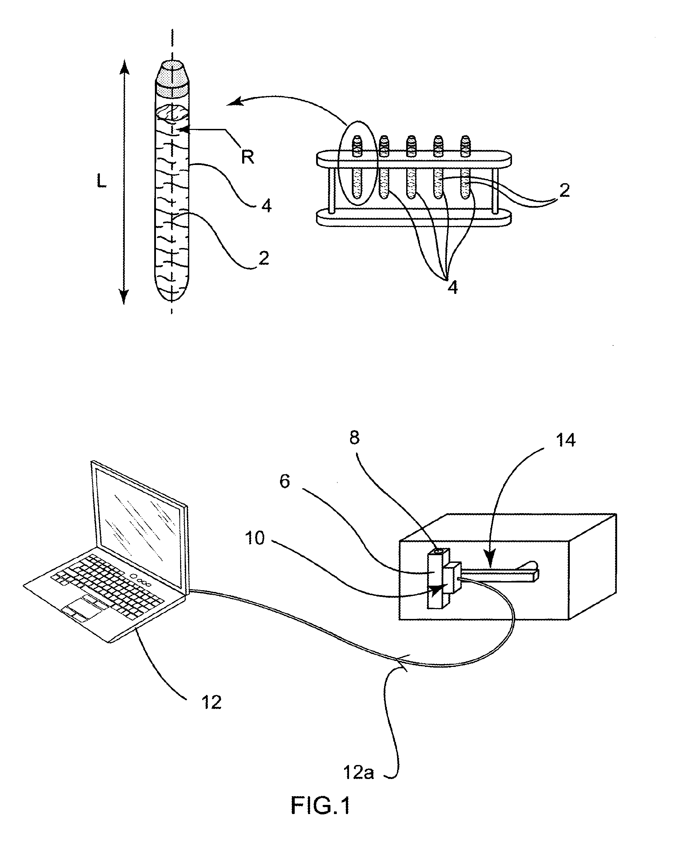

La figure 1 représente, en perspective, un exemple de réalisation d'un dispositif selon l'invention destiné à mesurer la vitesse d'agrégation d'une suspension sanguine 2 prélevée sur un patient à partir d'un tube de prélèvement 4. La suspension sanguine 2 est donc comprise dans le tube de prélèvement 4 et la mesure peut avantageusement être réalisée directement à partir de ce tube de prélèvement 4.

Ainsi, la mesure peut être réalisée directement au lit du patient après avoir réalisé le prélèvement. En outre, il n'est pas nécessaire de procéder à un quelconque transfert de la suspension sanguine 2 depuis le tube de prélèvement 4 et vers un conteneur spécifique. Les risques de contamination du personnel médical sont donc limités.

Il convient de noter que le tube de prélèvement 4, comme cela est la norme aujourd'hui, présente une forme sensiblement cylindrique s'étendant selon une direction longitudinale. Le tube de prélèvement 4 présente donc une section transversale sensiblement circulaire, de rayon R, ainsi qu'une longueur L dans la direction longitudinale.

Ce tube de prélèvement 4 pourrait être remplacé par tout autre type de conteneur analogue ou similaire pour autant qu'il soit au moins en partie transparent et capable de recevoir un milieu complexe et présentant une forme s'étendant sensiblement selon une direction longitudinale.

De la même façon, dans le cadre de cet exemple de réalisation, le milieu complexe est formé par une suspension sanguine 2. Toutefois, il pourrait également être envisagé de réaliser une mesure sur un milieu complexe différent dés lors que celui-ci est à même de passer d'un premier état d'agrégation à un deuxième état d'agrégation lors d'une étape préalable d'agitation telle que décrite ultérieurement.

En vue de mesurer le temps d'agrégation, le dispositif a pour objet d'analyser l'évolution de la lumière rétrodiffusée par la suspension sanguine 2 immédiatement après une étape préalable d'agitation.

Pour ce faire, le dispositif comporte un module 6 à l'intérieur duquel est définie une chambre 8 dans laquelle peut être inséré le tube de prélèvement 4. Cette chambre 8 est également sensiblement cylindrique et présentent une section transversale circulaire de rayon légèrement supérieur au rayon R de la section transversale du tube de prélèvement 4. Dés lors, la chambre 8 permet de recevoir et de supporter le tube de prélèvement 4.

Selon une réalisation avantageuse la surface intérieure de la chambre 8 est recouverte au moins en partie par un joint torique 8a ou bien d'une portion caoutchouteuse (non représentée) ayant pour fonction de maintenir le tube de prélèvement 4 à l'intérieur de la chambre 8 et dans une position fixe ou sensiblement fixe vis-à-vis de cette chambre 8 lorsque le module 6 est animé d'un mouvement d'agitation. Plus particulièrement, la géométrie de la chambre 8 dont le rayon est ajusté au rayon R du tube de prélèvement 4 et l'utilisation simultanée d'une portion caoutchouteuse permet de maintenir le tube de prélèvement 4 en position.

Il convient de signaler, toutefois, que la portion caoutchouteuse pourrait être remplacée par d'autres moyens de maintien analogues ou similaires. Plus particulièrement, la portion caoutchouteuse pourrait être remplacée par une vis de pression capable de comprimer le tube de prélèvement 4 à l'intérieur de la chambre 8 ou bien par une pièce extérieure susceptible de venir en appui sur l'extrémité découverte du tube de prélèvement 4. Cette portion caoutchouteuse pourrait alternativement être remplacée par tout autre moyen mécanique analogue ou similaire.

Le dispositif de la figure 1 comporte en outre des moyens de support (non représentés) d'un module de mesure 10 de la lumière rétrodiffusée par la suspension sanguine 2. Le module de mesure 10 est relié de manière à transmettre des signaux de mesure à une station de calcul 12 permettant de déterminer, à partir de signaux de mesure 12a émis par ce module de mesure 10, la vitesse d'agrégation de la suspension sanguine 2.

Le dispositif comporte en outre des moyens de mise en mouvement 14 aptes à imposer au module 6 un mouvement d'agitation comprenant au moins un déplacement de va-et-vient selon un vecteur directionnel. Selon l'invention, ce vecteur directionnel s'étend en proportion non négligeable selon la direction longitudinale du tube de prélèvement 4 lorsqu'il est maintenu en position à l'intérieur de la chambre 8, de manière à provoquer des contraintes de cisaillement dans la suspension sanguine 2 engendrant au moins partiellement la destruction des agrégats contenus dans ladite suspension sanguine 2. Cette destruction des agrégats permet d'aboutir à un état initial reproductible à partir duquel peuvent être établies les mesures permettant de déterminer les propriétés du milieu complexe 2.

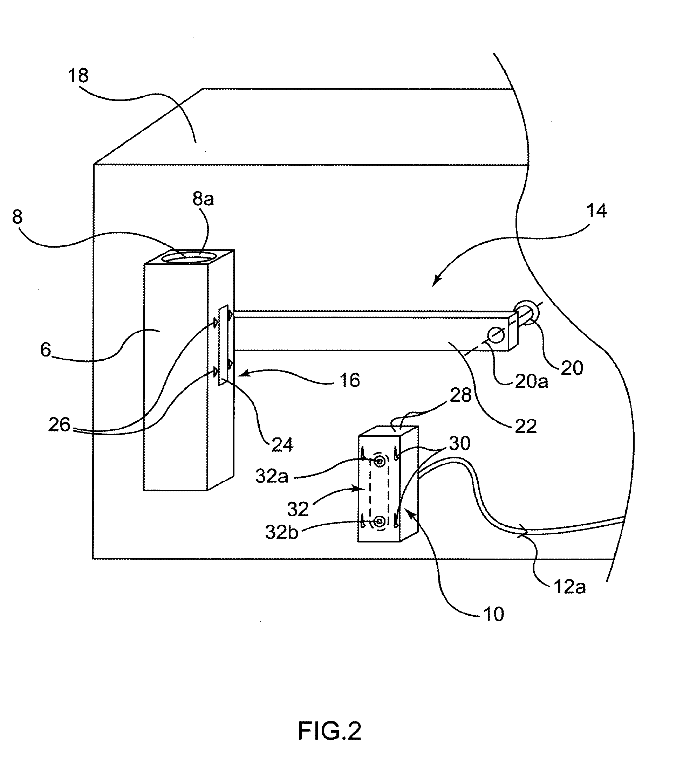

La figure 2 représente, en perspective et de façon plus détaillée, d'une part le module de mesure 10 et les moyens de support 16 de ce module de mesure 10 et, d'autre part, les moyens de mise en mouvement 14 du dispositif de la figure 1.

Il y a lieu de noter en premier lieu qu'une partie des moyens de mise en mouvement 14 sont intégrés à l'intérieur d'un coffret 18. Ce coffret 18 comporte un moteur (non représenté) à vitesse réglable. Selon différentes réalisations, ce moteur peut présenter n'importe quelle vitesse de rotation comprise entre 200 et 300 tours par minute. Le moteur est relié structurellement et fonctionnellement à un arbre de sortie 20 par l'intermédiaire d'un système mécanique - de bielle/manivelle, d'engrenages ou équivalents. Ce système mécanique permet ainsi d'ajuster l'amplitude et la vitesse de rotation de l'arbre de sortie 20 dont une extrémité est positionnée à l'extérieur du coffret 18.

L'arbre de sortie 20 est en outre relié par liaison fixe à un bras de support 22 supportant, également par liaison fixe, le module 6. Le bras de support 22 présente, selon un mode de réalisation avantageux, une longueur de 80 millimètres.

Alternativement, mais tout en conservant un encombrement limité, la longueur du bras de levier pourrait être comprise entre 50 et 200 millimètres et son déplacement en rotation pourrait être compris entre 10° et 90°.

D'autre part, le système mécanique est doté d'un capteur de position agencé de sorte que le bras de support 22 maintienne toujours le module 6 en position verticale lorsque le moteur est arrêté.

Il y a lieu de noter en second lieu, que l'exemple de réalisation de la figure 2 représente, de façon détaillée, des moyens de support 16 du module de mesure 10.

Plus particulièrement, selon cette réalisation le module 6 comporte une fenêtre transparente 24 s'étendant sensiblement selon la direction longitudinale du tube de prélèvement 4 ainsi que quatre encoches 26 positionnées autour de la fenêtre transparente 24.

Par ailleurs, le module de mesure 10 comporte un corps 28 s'étendant également dans une direction longitudinale, quatre ergots 30 aptes à coopérer avec les encoches 26 du module 6 ainsi qu'un ensemble optoélectronique 32 comprenant une source lumineuse d'émission 32a, telle qu'une diode émettrice infrarouge d'une longueur d'onde comprise ente 800 et 1000 nanomètres, et un détecteur optique 32b, tel qu'un capteur à photodiodes amplificatrices intégrées de type IPL (Integrated Photodiode Amplifiers).

Selon cette réalisation, la source lumineuse 32a présente une puissance de l'ordre de quelques milliwatts.

Les quatre ergots 30 du module de mesure 10 peuvent s'engager dans les encoches 26 du module 6 de sorte que le corps 28 soit maintenu en position vis-à-vis du module 6. Ainsi, les ergots 30 et les encoches 26 permettent d'associer structurellement et fonctionnellement le module de mesure 10 avec le module 6 de sorte que le mouvement d'agitation imposé au module 6 est également imposé au module de mesure 10. Dans cette position assemblée, la source lumineuse d'émission 32a est apte à émettre des rayons lumineux d'émission en direction de la suspension sanguine 2 de sorte à illuminer cette suspension sanguine 2 à travers la fenêtre transparente 24 du module 6. En outre, dans cette position assemblée, le détecteur optique 32b est apte à recevoir les rayons lumineux rétrodiffusés par la suspension sanguine 2 en réponse à l'illumination par les rayons lumineux d'émission.

Alternativement, les moyens de support du module de mesure 10 pourraient éventuellement être dissociés structurellement du module 6 de sorte que le module de mesure 10 soit en mouvement relatif vis-à-vis de du module 6 lorsque ledit module 6 est en mouvement d'agitation.

Par exemple, le module de mesure pourrait être dissocié structurellement du module 6 mais positionné en regard de la fenêtre transparente 24 de sorte que, malgré le mouvement du module 6 et donc de la fenêtre transparente 24 :
■ des rayons lumineux d'émission émis par la source lumineuse d'émission 32a puissent se propager à travers la fenêtre transparente 24 vers la suspension sanguine 2 et
■ des rayons lumineux rétrodiffusés par la suspension sanguine 2 puissent se propager au travers de la fenêtre transparente 24 vers le détecteur optique 32b.

Avantageusement, la source lumineuse d'émission 32a et le détecteur optique 32b sont placés l'un au dessus de l'autre et présente un angle d'orientation prédéterminé de l'ordre de 30° afin que les rayons lumineux d'émission issus de la source lumineuse d'émission 32a soient en grande partie rétrodiffusés vers le détecteur optique 32b.

La bissectrice de l'angle défini par cette source lumineuse d'émission 32a et ce détecteur optique 32b est, de préférence, positionné à mi-hauteur du tube de prélèvement 4.

L'ensemble optoélectronique 32 et, plus particulièrement ladite source lumineuse d'émission 32a et ledit détecteur optique 32b sont reliés par un câble souple à un boîtier d'alimentation et de contrôle de puissance. D'autre part, cet ensemble optoélectronique 32 est relié à la station de calcul 12 de manière à lui transmettre les signaux de mesure 12a recueillis lors de la phase de mesure.

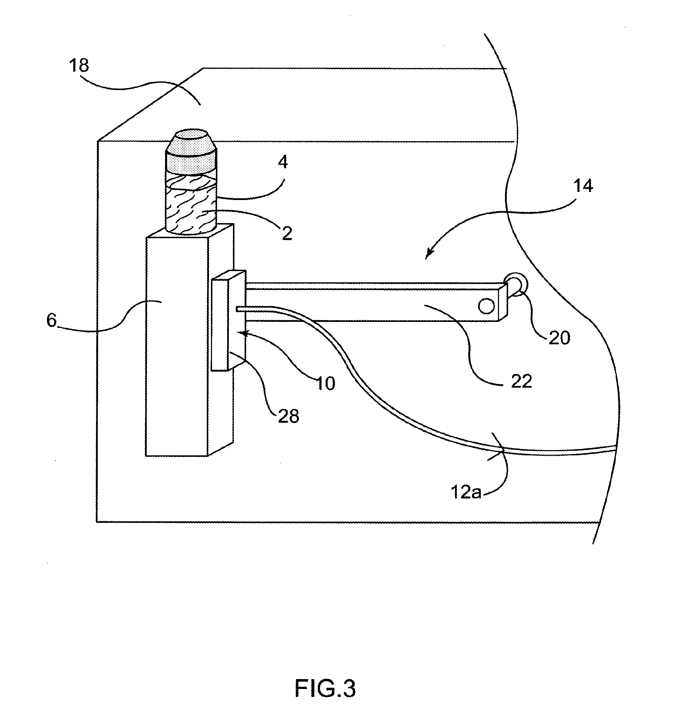

La figure 3 représente, en perspective et de façon détaillée, le dispositif de la figure 1 dans lequel le module de mesure 10 est structurellement associé au module 6 et le tube de prélèvement 4 est inséré dans la chambre 8 du module 6.

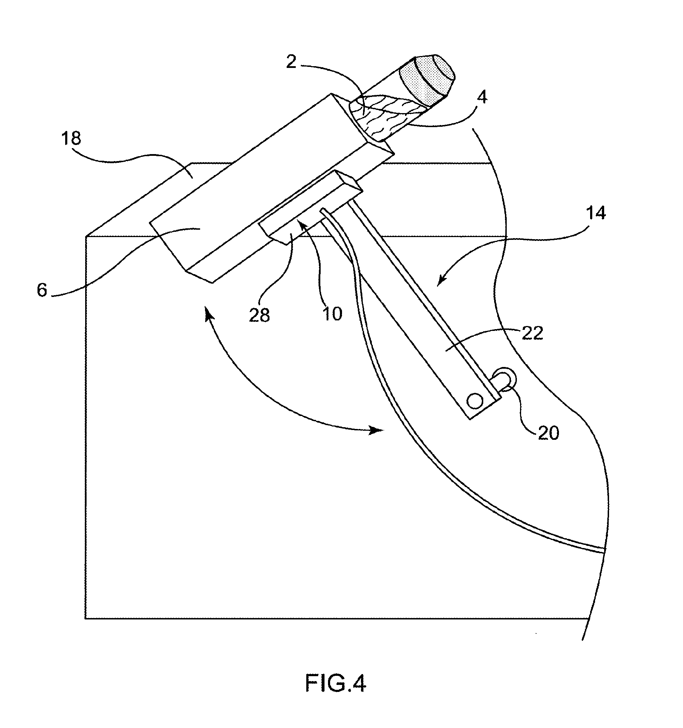

Vont maintenant être décrites plusieurs étapes mises en oeuvre dans un exemple de réalisation du procédé selon l'invention, à la lumière de la figure 4 qui représente, en perspective et de façon détaillée, le dispositif de la figure 1 lorsque celui-ci est en mouvement d'agitation.

Comme décrit précédemment, le procédé consiste à mesurer le temps d'agrégation de la suspension sanguine 2 par une analyse de l'évolution de la lumière rétrodiffusée par cette suspension sanguine lors d'une phase de mesure postérieure à une étape préalable d'agitation.

Lors de cette étape préalable d'agitation, le tube de prélèvement 4 contenant la suspension sanguine 2 est mis en mouvement. Plus particulièrement, la mise en marche du moteur entraîne la rotation de l'arbre de sortie ce qui permet de faire pivoter le bras de support 22 ainsi que le module 6 autour de l'axe de rotation 20a de l'arbre de sortie 20 dans un mouvement de va-et-vient.

Plus particulièrement, les moyens de mise en mouvement 14 imposent au module 6 un déplacement de va-et-vient selon un vecteur directionnel s'étendant en proportion non négligeable selon la direction longitudinale du tube de prélèvement 4, de manière à provoquer des contraintes de cisaillement dans la suspension sanguine 2 engendrant la destruction des agrégats. Ce mouvement d'agitation présente avantageusement une fréquence d'agitation d'environ 4 Hz, ce qui permet d'imposer des contraintes de cisaillement suffisamment élevées pour désagréger la majeure partie des agrégats de la suspension sanguine 2. Toutefois, une fréquence d'agitation comprise entre 2 et 6 hertz serait également envisageable et permettrait d'obtenir des résultats satisfaisants.

Apres une étape préalable d'agitation de 10 secondes environ, le moteur est arrêté.

Le module de mesure 10 permet alors de suivre l'évolution de la lumière rétrodiffusée par la suspension sanguine 2 dans un intervalle de temps d'environ 2 minutes. Les signaux de mesure 12a correspondant sont ensuite transmis à la station de calcul 12 qui, par l'intermédiaire d'une carte d'acquisition telle qu'une National Instrument DAQPad-12000 puis d'un programme de traitement de données, permet par une méthode bien connue de l'état de la technique de déduire le temps d'agrégation de la suspension sanguine 2.

Il convient de noter que selon la réalisation préalablement décrite, les moyens de mise en mouvement 14 imposent un mouvement d'agitation correspondant à une rotation du module 6 autour d'un axe.

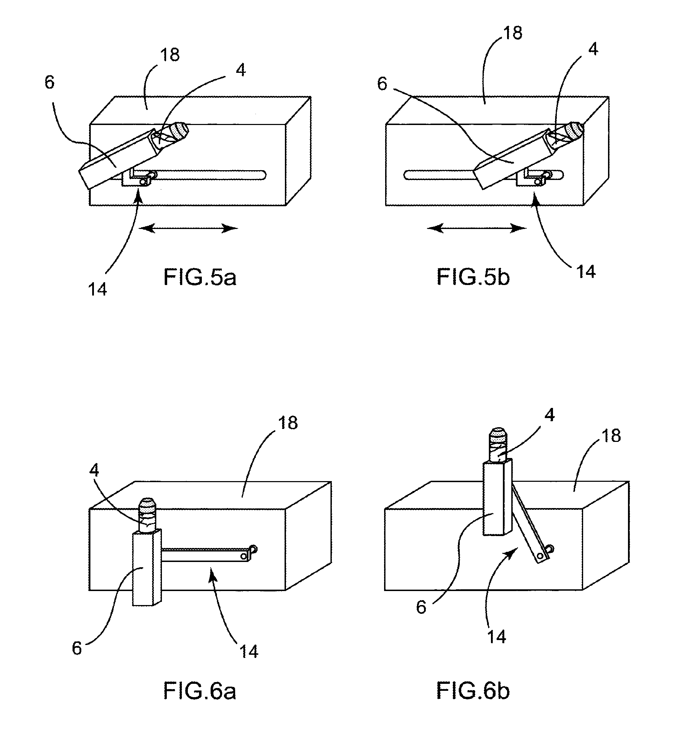

Toutefois, comme l'illustrent les figures 5a, 5b et 6a, 6b, d'autres mouvements comprenant au moins un déplacement de va-et-vient selon un vecteur directionnel s'étendant en proportion non négligeable selon la direction longitudinale du tube de prélèvement 4 pourraient être utilisés.

Plus particulièrement, comme l'illustrent les figure 5a et 5b, le dispositif selon l'invention par ailleurs semblable au mode de réalisation de la figure 1 pourrait comprendre des moyens de mise en mouvement 14 permettant d'obtenir un mouvement d'agitation correspondant à un déplacement de va-et-vient selon une translation unidirectionnelle.

Cette translation unidirectionnelle peut présenter un vecteur directionnel s'étendant sensiblement, mais pas totalement, dans la direction longitudinale du tube de prélèvement 4 lorsque celui-ci est en position à l'intérieur du module 6. Ainsi, il est possible d'obtenir des résultats satisfaisant même si le tube de prélèvement 4 est incliné vis-à-vis de la direction de translation des moyens de mis en mouvement 14.

Alternativement et tel qu'illustré sur les figure 6a et 6b, il serait également possible que le dispositif - par ailleurs semblable au mode de réalisation de la figure 1 - comprenne des moyens de mise en mouvement 14 permettant d'obtenir un mouvement d'agitation correspondant à un déplacement de va-et-vient selon une translation circulaire autour d'un axe de rotation 20a.

En pratique, il suffit que la composante du vecteur directionnel dans la direction longitudinale correspondant à la projection du vecteur directionnel dans la direction longitudinal du tube de prélèvement 4 lorsque celui-ci est en position dans le module 6 soit suffisamment importante pour désagréger les agrégats de la suspension sanguine 2. Cette composante longitudinale peut être supérieure à toute autre composante transversale s'étendant selon une direction perpendiculaire à la direction longitudinale susmentionnée.

Il convient de souligner que les modes de réalisations décrits ci-dessus concernent la mesure de la vitesse d'agrégation d'une suspension sanguine 2. Toutefois, le dispositif selon l'invention est également adapté pour réaliser une mesure du temps de sédimentation de la suspension sanguine 2 ou de tout autre milieu complexe.

A cet égard, le procédé de mesure de la vitesse de sédimentation diffère du procédé de mesure de la vitesse d'agrégation en ce que, postérieurement à la phase préalable d'agitation, l'analyse de l'évolution de la lumière rétrodiffusée par la suspension sanguine 2 s'étend dans un intervalle de temps d'environ une ou deux heures et non plus de deux minutes seulement. Les étapes postérieures à cette phase préalable d'agitation - et plus particulièrement les étapes d'analyse de la lumière rétrodiffusée par le milieu complexe - sont aujourd'hui bien connues de l'homme du métier.

Il convient également de noter que, pour réaliser une telle mesure de la vitesse de sédimentation, il est préférable d'utiliser une fenêtre s'étendant sensiblement selon toute la longueur du conteneur.

Il convient également d'ajouter que le dispositif de mesure selon l'invention peut, selon un mode de réalisation avantageux, permettre de mesurer simultanément le temps d'agrégation et la vitesse de sédimentation de la suspension sanguine 2.

Pour ce faire, une première série de rayons d'émission illumine la suspension sanguine 2 selon une première direction ; le temps d'agrégation est alors mesuré par analyse des rayons lumineux rétrodiffusés par la suspension sanguine 2. D'autre part, une deuxième série de rayons d'émission illumine la suspension sanguine 2 selon une deuxième direction ; le temps de sédimentation est alors mesurée par analyse des rayons traversants ou rétrodiffusés par ladite suspension sanguine 2.

## Revendications

1. Dispositif destiné à mesurer les propriétés d'un milieu complexe (2) par une analyse de l'évolution de la lumière rétrodiffusée et/ou transmise par le milieu complexe (2) lors d'une phase de mesure postérieure à une étape préalable d'agitation, ce milieu complexe (2) comprenant des agrégats et étant contenu dans un conteneur (4) s'étendant selon une direction longitudinale, le dispositif de mesure comportant :
■ des moyens de réception (8) aptes à recevoir et à supporter le conteneur (4) ;
■ des moyens de maintien (8,8a) aptes à maintenir ce conteneur (4) dans une position fixe ou sensiblement fixe vis-à-vis des moyens de réception (8) lorsque ces moyens de réception (8) sont animés d'un mouvement d'agitation provoquant la destruction des agrégats dudit milieu complexe (2) ;
■ des moyens de support (26) d'un module de mesure (10) de la lumière rétrodiffusée et/ou transmise par ledit milieu complexe (2), les moyens de support (26) étant :
o aptes à s'associer structurellement avec le module de mesure (10) de sorte à le supporter, et
o agencés vis-à-vis desdits moyens de réception (8) de manière à permettre au module de mesure (10) d'émettre des rayons lumineux d'émission de sorte à illuminer ledit milieu complexe (2) et de recevoir des rayons lumineux rétrodiffusés et/ou transmis par ledit milieu complexe (2) à tout moment de la phase de mesure lorsque ce module de mesure (10) est associé structurellement aux moyens de support (26) ;
**caractérisé en ce qu'**il comporte des moyens de mise en mouvement (14) :
■ aptes à imposer auxdits moyens de réception (8) un mouvement d'agitation comprenant au moins un déplacement de va-et-vient selon un vecteur directionnel s'étendant en proportion non négligeable selon la direction longitudinale,
■ de manière à provoquer des contraintes de cisaillement dans le milieu complexe (2) engendrant la destruction des agrégats dudit milieu complexe (2) lorsque ledit conteneur (4) est reçu et supporté par les moyens de réception (8).

2. Dispositif de mesure selon la revendication 1, dans lequel les moyens de mise en mouvement (14) sont aptes à imposer auxdits moyens de réception (8) un mouvement d'agitation comprenant au moins un déplacement de va-et-vient selon une translation unidirectionnelle.

3. Dispositif de mesure selon la revendication 1, dans lequel les moyens de mise en mouvement (14) sont aptes à imposer auxdits moyens de réception (6) un mouvement d'agitation comprenant au moins un déplacement de va-et-vient selon une translation circulaire autour d'un axe de rotation (20a).

4. Dispositif de mesure selon la revendication 1, dans lequel les moyens de mise en mouvement (14) sont aptes à imposer auxdits moyens de réception (6) un mouvement d'agitation comprenant au moins un déplacement de va-et-vient selon une rotation autour d'un axe de rotation (20a).

5. Dispositif selon la revendication 3 ou 4, dans lequel l'axe de rotation (20a) est positionné à une distance sensiblement comprise entre 50 et 200 millimètres du centre de gravité des moyens de réception (6).

6. Dispositif selon l'une quelconque des revendications 3 à 5, dans lequel le déplacement de va-et-vient autour de l'axe de rotation (20a) est sensiblement compris entre 10° et 90°.

7. Dispositif de mesure selon l'une quelconque des revendications 3 à 6, dans lequel les moyens de mise en mouvement (14) comprennent :
■ un moteur présentant un arbre de rotation,
■ un bras de levier (22) :
∘ coopérant avec l'arbre de rotation et relié aux moyens de réception (6)
∘ de sorte que les moyens de mise en mouvement (14) sont aptes à entraîner les moyens de réception (6) en déplacement autour de l'arbre de rotation.

8. Dispositif de mesure selon l'une quelconque des revendications 1 à 7, dans lequel les moyens de maintien (8,8a) sont formés par au moins un joint torique (8a) recouvrant une portion de la surface intérieure des moyens de réception (6).

9. Dispositif de mesure selon l'une quelconque des revendications 1 à 7, dans lequel les moyens de maintien (8,8a) sont formés par un pince ou une pièce de butée déformable ou non.

10. Dispositif de mesure selon l'une quelconque des revendications 1 à 7, comprenant le module de mesure (10) de la lumière rétrodiffusée et/ou transmise par ledit milieu complexe (2), et dans lequel ce module de mesure (10) de la lumière comporte :
■ une source lumineuse (32a) apte à émettre les rayons lumineux d'émission en direction dudit milieu complexe (2) de sorte à illuminer ledit milieu complexe (2),
■ un détecteur optique (32b) apte à recevoir les rayons lumineux rétrodiffusés et/ou transmis par ledit milieu complexe (2) en réponse à l'illumination dudit milieu complexe (2).

11. Dispositif de mesure selon la revendication 10, dans lequel la source lumineuse (32a) émet un rayonnement monochromatique.

12. Dispositif de mesure selon l'une quelconque des revendications 1 à 11, dans lequel les moyens de support (26) du module de mesure (10) sont associés structurellement et fonctionnellement aux moyens de réception (6) du conteneur (4) de sorte que le mouvement d'agitation imposé aux moyens de réception (6) est également imposé au module de mesure (10).

13. Dispositif de mesure selon l'une quelconque des revendications 1 à 11, dans lequel les moyens de support (26) du module de mesure (10) sont dissociés structurellement des moyens de réception (6) de sorte que ce module de mesure (10) est en mouvement relatif vis-à-vis de ces moyens de réception (6) lorsque lesdits moyens de réception (6) sont en mouvement d'agitation.

14. Dispositif de mesure selon l'une quelconque des revendications 1 à 13, dans lequel les moyens de réception (6) présentent une fenêtre (24) agencée vis-à-vis des moyens de support (26) de sorte que les rayons lumineux rétrodiffusés et les rayons lumineux d'émission sont aptes à traverser cette fenêtre (24) lors de la phase de mesure.

15. Procédé destiné à mesurer les propriétés d'un milieu complexe (2) par une analyse de l'évolution de la lumière rétrodiffusée et/ou transmise par le milieu complexe (2) lors d'une phase de mesure postérieure à une étape préalable d'agitation, ce milieu complexe (2) comprenant des agrégats et étant contenu dans un conteneur (4) s'étendant selon une direction longitudinale, le procédé comprenant une pluralité d'étapes successives durant lesquelles :
■ le conteneur (4) est reçu et supporté par des moyens de réception (6) ;
■ le conteneur (4) est maintenu dans une position fixe ou sensiblement fixe vis-à-vis des moyens de réception (6) ;
■ un ensemble optoélectronique (32) émet des rayons lumineux d'émission de sorte à illuminer le milieu complexe (2) et à recevoir des rayons lumineux rétrodiffusé et/ou transmis par ledit milieu complexe (2) ;
**caractérisé en ce que** l'étape préalable d'agitation du conteneur (4) consiste à :
■ mettre les moyens de réception (6) dans un mouvement d'agitation comprenant au moins un déplacement de va-et-vient selon un vecteur directionnel s'étendant en proportion non négligeable selon la direction longitudinale,
■ de manière à provoquer des contraintes de cisaillement dans le milieu complexe (2) engendrant la destruction des agrégats dudit milieu complexe (2) lorsque ledit conteneur (4) est reçu et supporté par les moyens de réception (6).

16. Procédé selon la revendication 15, dans lequel l'étape préalable d'agitation consiste à mettre les moyens de réception (6) et le conteneur (4) dans un mouvement d'agitation comprenant au moins un déplacement de va-et-vient selon une translation unidirectionnelle.

17. Procédé selon la revendication 15, dans lequel l'étape préalable d'agitation consiste à mettre les moyens de réception (6) et le conteneur (4) dans un mouvement d'agitation comprenant au moins un déplacement de va-et-vient selon une translation circulaire autour d'un axe de rotation (20a).

18. Procédé selon la revendication 15, dans lequel l'étape préalable d'agitation consiste à mettre les moyens de réception (6) et le conteneur (4) dans un mouvement d'agitation comprenant au moins un déplacement de va-et-vient selon une rotation autour d'un axe de rotation (20a).

19. Procédé selon la revendication 17 ou 18, dans lequel l'axe de rotation (20a) est positionné à une distance sensiblement comprise entre 50 et 200 millimètres du centre de gravité des moyens de réception (6).

20. Procédé selon l'une quelconque des revendications 17 à 19, dans lequel le déplacement de va-et-vient autour de l'axe de rotation (20a) est sensiblement compris entre 10° et 90°.

21. Procédé selon l'une quelconque des revendications 15 à 20, dans lequel le conteneur (4) est maintenu par un joint torique recouvrant une portion de la surface interne des moyens de réception (6).

22. Procédé selon l'une quelconque des revendications 15 à 20, dans lequel le conteneur (4) est maintenu par un pince ou une pièce de butée déformable ou non.

23. Procédé selon l'une quelconque des revendications 15 à 22, dans lequel, durant la phase de mesure :
■ une source lumineuse (32a) émet les rayons lumineux d'émission en direction dudit milieu complexe (2) de sorte à illuminer ledit milieu complexe (2),
■ un détecteur optique (32b) reçoit les rayons lumineux rétrodiffusés et/ou transmis par ledit milieu complexe (2) en réponse à l'illumination dudit milieu complexe (2).

24. Procédé selon l'une quelconque des revendications 23, dans lequel la source lumineuse (32a) émet un rayonnement monochromatique.

25. Procédé selon l'une quelconque des revendications 15 à 24, dans lequel les moyens de support (26) du module de mesure (10) sont associés structurellement et fonctionnellement aux moyens de réception (6) du conteneur (4) de sorte que le mouvement d'agitation imposé aux moyens de réception (6) est également imposé au module de mesure (10).

26. Procédé selon l'une quelconque des revendications 15 à 24, dans lequel les moyens de support du module de mesure (10) sont dissociés structurellement des moyens de réception (6) de sorte que ces moyens de support sont en mouvement relatif vis-à-vis de ces moyens de réception (6) lorsque lesdits moyens de réception (6) sont en mouvement d'agitation.

27. Procédé selon l'une quelconque des revendications 15 à 26, dans lequel les moyens de réception (6) présentent une fenêtre (24) agencée vis-à-vis des moyens de support (26) du module de mesure (10) de sorte que les rayons lumineux d'émission et les rayons lumineux rétrodiffusés traversent cette fenêtre (24) lors de la phase de mesure.

28. Procédé selon l'une quelconque des revendications 15 à 27, dans lequel le module de mesure (10) transmettent des données collectées durant la phase de mesure à une unité d'analyse (12) déportée structurellement ou non.

29. Procédé selon la revendication 28, dans lequel l'unité d'analyse (12) utilise les données collectées pour calculer le temps de sédimentation dudit milieu complexe (2) postérieurement à la phase de mesure.

## Patentansprüche

1. Vorrichtung, die zur Messung der Eigenschaften eines komplexen Milieus (2) durch eine Analyse der Entwicklung des Licht, das durch das komplexe Milieu (2) bei einer Messphase nach einer vorherigen Schüttelstufe zurückgeworfen und / oder übertrafen wird, bestimmt ist, wobei dieses komplexe Milieu (2) Aggregate umfasst und in einem Behälter (4) entfalten ist, der sich gemäß einer Längsrichtung erstreckt, wobei die Messvorrichtung umfasst:
* Aufnahmemittel (8), die geeignet sind, den Behälter (4) auszunehmen rund zu tragen;
* Festhaltemittel (8, 8a), die geeignet sind, dienen Behälter (4) in einer festen oder deutlich festen Postition gegenüber den Aufnahmemitteln (8) zu halten, wenn diese Aufnahmemittel (8) durch eine Schüttelbewegung bewegt sind, die die Zerstörung der Aggregate des genannten komplexen Milieus (2) hervorruft
* Trägermittel (26) eines Messmittels (10) des durch das genannte komplexe Milieu (2) zurückgestrahlten und / oder übertragenen Lichts, wobei die Trägermittel (26):
O geeignet sind, sich strukturell mit dem Messmodul (10) derart zu verfinden, dass es getragen wird, und
∘ gegenüber den Aufnahmemitteln (8) derart angeordnet sind, dass dem Messmodul (10) die Ausgabe von Ausgabe-Lichtstrahlen derart ermöglicht wird, dass das genannte komplexe Milieu (2) beleuchtet wird und Lichtstrahlen aufgenommen werden, die von dem genannten komplexen Milieu (2) zu jedem Zeitpunkt der Messphase zurückgeworfen und / oder übertragen werden, wenn dieses Messmodul (10) strukturell mit den Trägermitteln (26) verbunden ist;
**dadurch gekennzeichnet, dass** es Mittel zum In-Bewegung-Setzen (14) umfasst:
* die geeignet sind, den Aufnahmemitteln (8) eine Schüttelbewegung aufzuzwingen, die wenigstens eine Hin-und Her-Verschiebung gemäß einem Richtungsvektor umfasst, der sich in nicht unerheblichen Verhältnis gemäß der Längsrichtung erstreckt,
* derart, dass Abscherspannungen in dem komplexen Milieu (2) hervorgerufen werden, die die Zerstörung der Aggregate des genannten komplexen Milieus (2) nach sich ziehen, wenn der genannte Behälter (4) durch Aufnahmemittel (8) aufgenommen und getragen ist.

2. Messvorrichtung gemäß Anspruch 1, bei der die Mittel zum In-Bewegung-Setzen (14) geeignet sind, den genannten Aufnahmemitteln (8) eine Schüttelbewegung aufzuzwingen, die wenigstens eine Hin-und-Her-Verschiebung gemäß einer Translation in einer Richtung umfasst.

3. Messvorrichtung gemäß Anspruch 1, bei der die Mittel zum In-Bewegung-Setzen (14) geeignet sind, den genannten Aufnahmemittel (6) eine Schüttelbewegung aufzuzwingen, die wenigstens eine Hin-und-Her-Verschiebung gemäß einer kreisförmigen Translation um eine Rotationsachse (20a) umfasst.

4. Messvorrichtung gemäß Anspruch 1, bei der die Mittel zum In-Bewegung-Setzen(14) geeignet sind, den genannten Aufnahmemitteln (6) eine Schüttelbewegung aufzuzwingen, die wenigstens eine Hin-und-Her-Bewegung gemäß einer Rotation um eine Rotationsachse (20a) umfasst.

5. Vorrichtung gemäß Anspruch 3 oder 4, bei der die Rotationsachse (20a) in einer deutlich zwischen 50 und 200 Millimeter vom Schwerpunkt der Aufnahmemittel (6) inbegriffenen Entfernung positioniert ist.

6. Vorrichtung gemäß Anspruch 3 bis 5, bei der die Hin-und-Her-Verschiebung um die Rotationsachse (20a) deutlich zwischen 10° und 90° inbegriffen ist.

7. Messvorrichtung gemäß Anspruch 3 bis 6, bei der die Mittel zum In-Bewegung-Setzen (14) umfassen:
* einen Motor, der eine Rotationswelle aufweist,
* einen Hebelarm (22),
∘ der mit der Rotationswelle zusammenwirkt und mit den Aufnahmemitteln (6) verbunden ist,
∘ derart, dass die Mittel zum In-Bewegung-Setzen (14) geeignet sind, Aufnahmemittel (6) in Verschiebung um die Rotationswelle anzutreiben.

8. Messvorrichtung gemäß Anspruch 1 bis 7, bei der die Festhaltemittel (8, 8a) durch wenigstens einen O-Ring (8a) geformt sind, der einen Abschnitt der inneren Fläche der Aufnahmemittel (6) abdeckt.

9. Messvorrichtung gemäß Anspruch 1 bis 7, bei der die Haltemittel (8, 8a) durch eine Klemme oder ein verformbares oder nicht verformbares Anschlagsteil geformt sind.

10. Messvorrichtung gemäß Anspruch 1 bis 7, umfassend das Messmodul (10) des Lichts, das durch das genannte komplexe Milieu (2) zurückgeworfen und / oder übertragen wird, und bei der dieses Messmodul (10) des Lichts umfasst:
* eine Lichtquelle (32a), die geeignet ist, die Ausgabe-Lichtstrahlen in Richtung des komplexen Milieus (2) derart auszugeben, dass genannte komplexe Milieu (2) beleuchtet wird,
* einen optischen Geber (32b), der geeignet ist, die Lichtstrahlen zu empfangen, die von dem genannten komplexen Milieu (2) als Antwort auf die Beleuchtung des genannten komplexen Milieus (2) zurückgeworfen und / oder übertragen werden.

11. Messvorrichtung gemäß Anspruch 10, bei der die Lichtquelle (32a) eine einfarbige Strahlung ausgift.

12. Messvorrichtung gemäß Anspruch 1 bis 11, bei der die Trägermittel (26) des Messmoduls (10) strukturell und funktional den Aufnahmemitteln (6) des Behälters (4) derart zugeordnet sind, dass die den Aufnahmemitten (6) aufgezwungen Schüttelbewegung ebenfalls dem Messmodul (10) aufgezwungen wird.

13. Messvorrichtung gemäß Anspruch 1 bis 11, bei der die Trägermittel (26) des Messmoduls (10) strukturell von den Aufnahmemittel (6) derart getrennt sind, dass dieses Messmodul (10) sich in einer relativen Bewegung gegenüber diesem Aufnahmemitteln (6) befindet, wenn die genanntem Aufnahmemittel (6) sich in einer Schüttelbewegung befinden.

14. Messvorrichtung gemäß Anspruch 1 bis 13, bei der die Aufnahmemittel (6) ein Fenster (24) aufweisen, das gegenüber den Trägermittel (26) derart ungeordnet ist, dass die zurückgeworfenen Lichtstrahlen und die Ausgabe-Lichtstrahlen geeignet sind, dieses Fenster (24) in der Messphase zu durchquerten.

15. Verfahren, das zur Messung der Eigenschaften eines komplexen Milieus (2) durch eine Analyse der Entwicklung des Lichts bestimmt ist, das durch das komplexe Milieu (2) bei einer Messphase nach einer vorherigem Schüttelphase zurückgeworfen und / oder übertragen wird, wobei dieses komplexe Milieu (2) Aggregate umfasst und in einem Behälter (4) enthalten ist, der sich gemäß einer Längsrichtung erstreckt, wobei das Verfahren eine Vielzahl von sukzessiven Stufen umfasst, in denen:
* der Behälter (4) durch Aufnahmemittel (6) aufgenommen und getragen ist;
* der Behälter (4) in einer festen oder deutlich festen Position gegenüber Aufnahmemitteln (6) gehalten ist;
* eine opto-elektrische Gruppe (32) Ausgabe-Lichtstrahlen derart ausgibt, dass das komplexe Milieu (2) beleuchtet wird und von dem genannten komplexen Milieu (2) zurückgeworfene und / oder übertragene Lichtstrahlen aufgenommen werden;
**dadurch gekennzeichnet, dass** die vorherige Schüttelstufe des Behälters (4) darin besteht,
* die Aufnahmemittel (6) in eine Schüttelbewegung zu verbringen, die wenigstens eine Hin-und-Her-Verschiebung gemäß einem Richtungsvektor umfasst, der sich in einem nicht unerheblichen Verhältnis gemäß der Längsrichtung erstreckt,
* Abscherspannungen derart in dem komplexen Milieu (2) hervorzurufen, dass die Zerstörung Aggregate des genannten komplexen Milieus (2) hervorgerufen wird, wenn der genannte Behälter (4) durch die Aufnahmemittel (6) aufgenommen und getragen ist.

16. Verfahren gemäß Anspruch 15, bei dem die vorherige Schüttelstufe darin besteht, die aufnahmemittel (6) rund den Behälter (4) in eine Schüttelbewegung zu versetzen, die wenigsten eine Hin-und-Her-Verschiebung gemäß einer Translation in eine Richtung umfasst.

17. Verfahren gemäß Anspruch 15, bei dem die vorherige Schüttelphase darin besteht, die Aufnahmemittel (6) und den Behälter (4) in eine Schüttelbewegung zu versetzten, die wenigstens eine Hin-und-Her-Verschiebung gemäß einer kreisförmigem Translation um eine Rotationsachse (20a) umfasst.

18. Verfahren gemäß Anspruch 15, bei dem die vorherige Schüttelphase darin besteht, die Aufnahmemittel (6) und den Behälter (4) in eine Schüttelbewegung zu versetzten, die wenigsten eine Hin-und-Her-Verschiebung gemäß einer Rotation um eine Rotationsachse (20a) umfasst.

19. Verfahren gemäß Anspruch 17 oder 18, bei dem die Rotationsachse (20a) in einer deutlich zwischen 50 und 200 Millimeter inbegriffener Entfernung vom Scherpunkt der Aufnahmemittel (6) entfernt ist.

20. Verfahren gemäß Anspruch 17 bis 19, bei dem die Hin-und-Her-Verschiebung um die Rotationsachse (20a) deutlich zwischen 10° und 90° inbegriffen ist.

21. Verfahren gemäß Anspruch 15 bis 20, bei dem der Behälter (4) durch einen O-Ring gehalten wird, der einen Abschnitt der inneren Fläche der Aufnahmemittel (6) abdeckt.

22. Vorfahren gemäß Anspruch 15 bis 20, bei dem der Behälter (4) durch eine Klemme oder ein verformbares oder nicht verformbares Anschlagskeil gehalten wird.

23. Vorfahren gemäß Anspruch 15 bis 22, bei dem wähnend der Messphase:
* eine Lichtquelle (32a) Ausgabe-Lichtstrahlen in Richtung des genannten komplexen Milieus (2) derart ausgibt, dass das genannte komplexe Milieu (2) beleuchtet wird,
* ein optischer Geber (32b) die Lichtstrahlen empfängt, die von dem genannten komplexen Milieu (2) als Antwort auf die Beleuchtung des genannten komplexen Milieus (2) zurückgeworfen und / oder übertrafen werden.

24. Vorfahren gemäß Anspruch 23, bei dem die Lichtquelle (32a) eine einfarbige Strahlung aufgibt.

25. Vorfahren gemäß Anspruch 15 bis 24, bei dem die Trägermittel (26) des Messmoduls (10) strukturell und funktional den Aufnahmemitteln (6) des Behälters (4) derart zugeordnet sind, dass die den Aufnahmemitteln (6) auferlegte Schüttelbewegung dem Messmodul (10) ebenfalls auferlegt wird.

26. Verfahren gemäß Anspruch 15 bis 24, bei dem die Trägermittel des Messmoduls (10) strukturell von den Aufnahmemitteln (6) derart gelöst sind, dass diese Trägermittel sich in relativer Bewegung gegenüber diesen Aufnahmemitteln (6) befinden, wenn die genannten Aufnahmemittel (6) sich in einer Schüttelbewegung befinden.

27. Verfahren gemäß Anspruch 15 bis 26, bei dem die Aufnahmemittel (6) ein Fenster (24) aufweisen, das gegenüber den Trägermitteln (26) des Messmoduls (10) derart angeordnet ist, dass die Ausgabe-Lichtstrahlen und die zurückgeworfenen Lichtstrahlen dieses Fenster (24) in der Messphase durchqueren.

28. Verfahren gemäß Anspruch 15 bis 27, bei dem das Messmodul (10) während einer Messphase erfasste Daten auf ein strukturell oder nicht strukturell versetztes Analysegerät (12) überträgt.

29. Verfahren gemäß Anspruch 28, bei dem das Analysegerät (12) die erfassten Daten verwendet, um die Sedimentationszeit des genannten komplexen Milieus (2) nach der Messphase zu berechnen.

## Claims

1. A device intended to measure the properties of a complex medium (2) by analysing the variation in the light backscattered and/or transmitted by the complex medium (2) during a measuring step following a prior stirring step, with said complex medium (2) comprising aggregates and being contained in a container (4) extending in a longitudinal direction, with the measuring device comprising:
* receiving means (8) able to receive and to support the container (4);
* holding means (8, 8a) able to hold such container (4) in a fixed or substantially fixed position with respect to the receiving means (8) when such receiving means (8) are subjected to a stirring movement generating the destruction of the aggregates in said complex medium (2);
* means (26) for supporting the module (10) for measuring the light backscattered and/or transmitted by said complex medium (2), with the supporting means (26) being:
∘ able to structurally associate with the measuring module (10) so as to support same, and
∘ so arranged with respect to said receiving means (8) as to enable the measuring module (10) to emit emission light rays so as to illuminate said complex medium (2) and to receive light rays backscattered and/or transmitted by said complex medium (2) at any time of the measuring step when such measuring module (10) is structurally associated with the supporting means (26);
**characterized in that** it comprises movement means (14):
* capable of subjecting said receiving means (8) to a stirring movement comprising at least a back-and-forth movement along a directional vector extending by a not insignificant amount along the longitudinal direction,
* so as to generate shear stresses in the complex medium (2), thereby destroying aggregates in said complex medium (2) when said container (4) is received and supported by the receiving means (8).

2. A measuring device according to claim 1, wherein the movement means (14) are able to subject said receiving means (8) to a stirring movement comprising at least a back-and-forth movement along a one-way translation.

3. A measuring device according to claim 1, wherein the movement means (14) are able to subject said receiving means (6) to a stirring movement comprising at least a back-and-forth movement along a circular translation about an axis of rotation (20a).

4. A measuring device according to claim 1, wherein the movement means (14) are able to subject said receiving means (6) to a stirring movement comprising at least a back-and-forth movement along a rotation about an axis of rotation (20a).

5. A device according to claim 3 or 4, wherein the axis of rotation (20a) is positioned at a distance substantially ranging from 50 to 200 millimetres from the centre of gravity of the receiving means (6).

6. A device according to any one of claims 3 to 5, wherein the back-and-forth movement about the axis of rotation (20a) is substantially ranging from 10° to 90°.

7. A measuring device according to any one of claims 3 to 6, wherein the movement means (14) comprise:
* a motor having a rotating shaft,
* a lever arm (22):
∘ cooperating with the rotating shaft and connected to the receiving means (6)
∘ so that the movement means (14) are able to drive the receiving means (6) so that they move about the rotating shaft.

8. A measuring device according to any one of claims 1 to 7, wherein the holding means (8, 8a) are formed by at least one O-ring (8a) covering a portion of the inner surface of the receiving means (6).

9. A measuring device according to any one of claims 1 to 7, wherein the holding means (8, 8a) are formed by a deformable or not deformable clamp or stop.

10. A measuring device according to any one of claims 1 to 7, comprising the module for measuring (10) the light backscattered and/or transmitted by said complex medium (2), and wherein such light measuring module (10) comprises:
* a light source (32a) able to emit the emission light rays toward said complex medium (2) so as to illuminate said complex medium (2),
* an optimal detector (32b) able to receive the light rays backscattered and/or transmitted by said complex medium (2) in response to the illumination of said complex medium (2).

11. A measuring device according to claim 10, wherein the light source (32a) emits a monochromatic radiation.

12. A measuring device according to any one of claims 1 to 11, wherein the means (26) for supporting the measuring module (10) are structurally and functionally associated with the means (6) for receiving the container (4) so that the stirring movement which the receiving means (6) is subjected to is also imparted to the measuring module (10).

13. A measuring device according to any one of claims 1 to 11, wherein the supporting means (26) of the measuring module (10) are structurally separated from the receiving means (6) so that this measuring module (10) is in relative movement with respect to such receiving means (6) when said receiving means (6) are subjected to a stirring movement.

14. A measuring device according to any one of claims 1 to 13, wherein the receiving means (6) are provided with an aperture (24) so arranged with respect to the supporting means (26) that the backscattered light rays and the emitted light rays are able to go through the aperture (24) during the measuring step.

15. A method intended to measure the properties of a complex medium (2) by analysing the variation in the light backscattered and/or transmitted by the complex medium (2) during a measuring step following a prior stirring step, with such complex medium (2) comprising aggregates and being contained in a container (4) extending in a longitudinal direction, with the method comprising a plurality of successive steps during which:
* the container (4) is received and supported by receiving means (6);
* the container (4) is held in a fixed or substantially fixed position with respect to the receiving means (6)
* an optoelectronic assembly (32) emits emission light rays so as to illuminate the complex medium (2) and to receive light rays backscattered and/or transmitted by said complex medium (2);
**characterized in that** the prior step of stirring the container (4) consists in:
* subjecting the receiving means (6) to a stirring movement comprising at least a back-and-forth movement along a directional vector extending by a not insignificant amount along the longitudinal direction,
* so as to generate shear stresses in the complex medium (2), thereby destroying aggregates in said complex medium (2) when said container (4) is received and supported by the receiving means (6).

16. A method according to claim 15, wherein the prior stirring step consists in subjecting the receiving means (6) and the container (4) to a stirring movement comprising at least a back-and-forth movement along a one-way translation.

17. A method according to claim 15, wherein the prior stirring step consists in subjecting the receiving means (6) and the container (4) to a stirring movement comprising at least a back-and-forth movement along a circular translation about an axis of rotation (20a).

18. A method according to claim 15, wherein the prior stirring step consists in subjecting the receiving means (6) and the container (4) to a stirring movement comprising at least a back-and-forth movement along a rotation about an axis of rotation (20a).

19. A method according to claim 17 or 18, wherein the axis of rotation (20a) is positioned at a distance substantially ranging from 50 to 200 millimetres from the centre of gravity of the receiving means (6).

20. A method according to any one of claims 17 to 19, wherein the back-and-forth movement about the axis of rotation (20a) is substantially ranging from 10° to 90°.

21. A method according to any one of claims 15 to 20, wherein the container (4) is held by an O-ring covering a portion of the inner surface of the receiving means (6).

22. A method according to any one of claims 15 to 20, wherein the container (4) is held by a deformable or not deformable clamp or stop.

23. A method according to any one of claims 15 to 22, wherein, during the measuring step:
* a light source (32a) emits the emission light rays toward said complex medium (2) so as to illuminate said complex medium (2),
* an optical detector (32b) receives the light rays backscattered and/or transmitted by said complex medium (2) in response to the illumination of said complex medium (2).

24. A method according to claim 23, wherein the light source (32a) emits a monochromatic radiation.

25. A method according to any one of claims 15 to 24, wherein the means for supporting (26) the measuring module (10) are structurally and functionally associated with the means for receiving (6) the container (4), so that the stirring movement which the receiving means (6) subjected to is also imparted to the measuring module (10).

26. A method according to any one of claims 15 to 24, wherein the means for supporting the measuring module (10) are structurally separated from the receiving means (6) so that such supporting means are in relative movement with respect to such receiving means (6) when said receiving means (6) are subjected to a stirring movement.

27. A method according to any one of claims 15 to 26, wherein the receiving means (6) have an aperture (24) so arranged with respect to the means for supporting (26) the measuring module (10) that the emission light rays and the backscattered light rays go through such aperture (24) during the measuring step.

28. A method according to any one of claims 15 to 27, wherein the measuring module (10) transmits data collected during the measuring step to a structurally shifted or not shifted analyzing unit (12).

29. A method according to claim 28, wherein the analyzing unit (12) uses the collected data to calculate the time for the sedimentation of said complex medium (2) after measuring step.
